## Europäisches Patentamt

## European Patent Office

## Office européen des brevets

(11) Veröffentlichungsnummer: **0 005 293**
**B1**

(12) **EUROPÄISCHE PATENTSCHRIFT**

(45) Veröffentlichungstag der Patentschrift:
05.05.82

(21) Anmeldenummer: **79200199.2**

(22) Anmeldetag: **21.04.79**

(51) Int. Cl.³: **C 07 C 45/37**, C 07 C 49/403

54 **Verfahren und Vorrichtung zur Herstellung von Cyclohexanon.**

(30) Priorität: **29.04.78 NL 7804667**

(43) Veröffentlichungstag der Anmeldung:
**14.11.79 Patentblatt 79/23**

(45) Bekanntmachung des Hinweises auf die Patenterteilung:
**05.05.82 Patentblatt 82/18**

(84) Benannte Vertragsstaaten:
**BE DE FR GB IT NL**

(56) Entgegenhaltungen:
**DE-A-1 150 070**
**FR-A-1 033 694**
**GB-A-939 613**

(73) Patentinhaber: **STAMICARBON B.V., Postbus 10,
NL-6160 MC Geleen (NL)**

(72) Erfinder: **Van De Mond, Theodorus Johannes,
Oranjelaan 10, NL-6166 BR Geleen (NL)**
Erfinder: **Delahaye, Hubertus Johannes Aloysius,
Spekhouwerstraat 63, NL-6367 TT Voerendaal (NL)**

(74) Vertreter: **Hoogstraten, Willem Cornelis Roeland et al,
OCTROOIBUREAU DSM Postbus 9, NL-6160 MA Geleen
(NL)**

ACTORUM AG.

## Verfahren und Vorrichtung zur Herstellung von Cyclohexanon

Die Erfindung betrifft ein Verfahren zur Herstellung von Cyclohexanon, bei dem Benzol in der Gasphase zu Cyclohexan hydriert, das Cyclohexan in der flüssigen Phase mit Sauerstoff oder einem sauerstoffhaltigen Gas zu einem cyclohexanolhaltigen Gemisch oxidiert und das auf diese Weise erhaltene Cyclohexanol katalytisch zu Cyclohexanon dehydriert wird. Dabei fällt als Nebenprodukt Wasserstoffgas an, das jedoch infolge der darin enthaltenen organischen Verunreinigungen für chemische Synthesen im allgemeinen ungeeignet ist. Sogar nach Kühlung, um die Verunreinigungen möglichst weitgehend auszukondensieren, ist das Wasserstoffgas nur als Heizgas mit einem besonders niedrigen Heizwert zu verwenden.

Aus der britischen Patentschrift 939 613 ist ein Verfahren zur Herstellung von Phenol (nicht von Cyclohexanon) bekannt, bei dem Benzol zu Cyclohexan hydriert, das Cyclohexan zu einem Gemisch von Cyclohexanol und Cyclohexanon oxidiert, dieses Gemisch zu Phenol und Wasserstoff dehydriert und der Wasserstoff in der Hydrierungsstufe verwendet wird. Bei diesem bekannten Verfahren ist es nicht wirtschaftlich möglich, die Hydrierung des Benzols in der Gasphase auszuführen, insbesondere nicht mit einem Metall aus der Platingruppe als Katalysator, weil der Katalysator dann sehr schnell seine Wirksamkeit verliert. Wahrscheinlich ist dies auf die Anwesenheit von organischen Verunreinigungen in dem Wasserstoffgas, das aus der Cyclohexanoldehydrierungsstufe stammt, zurückzuführen. Dennoch ist es äusserst erwünscht, die Hydrierung des Benzols in der Gasphase, und vorzugsweise mit einem Metall aus der Platingruppe als Katalysator, auszuführen, weil dabei eine grosse Reaktionsgeschwindigkeit in Kombination mit einer hohen Ausbeute an Cyclohexan erreicht werden kann und auch schwefelhaltiges Benzol sich leicht verarbeiten lässt.

Aufgabe der vorliegenden Erfindung ist ein Verfahren zur Herstellung von Cyclohexanon, bei dem für das im Grunde wertlose Nebenprodukt, verunreinigtes Wasserstoffgas, eine sinnvolle Verwendungsmöglichkeit gefunden wird.

Nach der vorliegenden Erfindung wird Cyclohexanon dadurch hergestellt, dass in einer Hydrierungszone Benzol in der Gasphase zu Cyclohexan hydriert wird, das Cyclohexan in einer Oxidationszone in der flüssigen Phase mit einem molekularen Sauerstoff enthaltenden Gas zu einem cyclohexanolhaltigen Gemisch oxidiert wird, das auf diese Weise erhaltene Cyclohexanol in einer Dehydrierungszone katalytisch zu Cyclohexanon und Wasserstoff dehydriert wird und Cyclohexanon und unumgesetztes Cyclohexanol abgetrennt werden, welches Verfahren dadurch gekennzeichnet wird, dass das zurückbleibende hauptsächlich aus Wasserstoff bestehende Gasgemisch mit Cyclohexan oder Benzol ausgewaschen und das Gas anschliessend in die Hydrierungszone eingeleitet wird.

Das erfindungsgemässe Verfahren bietet erhebliche Ersparnisse an Wasserstoffgas bei der Hydrierung des Benzols unter Beibehaltung einer langen Standzeit des Hydrierungskatalysators, einer hohen Reaktionsgeschwindigkeit und einer Hydrierung mit hoher Ausbeute. Prozessfremde Stoffe werden nicht in das System eingeleitet. Die Waschflüssigkeit kann auf einfache Weise, z.B. mittels Destillation, wieder gereinigt werden. Ausserdem werden Verluste an Cyclohexanon und Cyclohexanol über den abgelassenen Wasserstoffablass vermieden.

Die Hydrierung von Benzol in der Gasphase wird vorzugsweise mit einem Metall aus der Platingruppe als Katalysator ausgeführt. Zu diesen Metallen gehören Platin selbst, das vorzugsweise verwendet wird, und weiter Osmium, Iridium, Ruthenium, Rhodium und Palladium. Auch können andere Katalysatoren, z.B. Nickel, verwendet werden. Das Metall befindet sich vorzugsweise auf einem Träger, vorzugsweise Aluminiumoxid, obwohl auch andere Träger, wie Silicagel oder Kieselgur, verwendet werden können. Die Hydrierung kann bei einer Temperatur von 150 bis 400°C und einem Druck von 0,5 bis 5 mPa erfolgen, obwohl für Temperatur und Druck auch höhere und niedrigere Werte benutzt werden können. Eine sehr gut geeignete Ausführungsform wird in der britischen Patentschrift 1 104 275 beschrieben.

Die Oxidation des Cyclohexans kann auf jede bekannte Weise, in Gegenwart oder Abwesenheit eines Katalysators z.B. einer Übergangsmetallverbindung, und mit oder ohne Isolierung des Zwischenprodukts Cyclohexylhydroperoxid, ausgeführt werden. Der Kürze halber wird auf die Berichte des Stanford Research Institute Nr. 3 (1965), 3A (1971), 7 (1965), 307–319 und 7A (1968), 87–103 und der amerikanischen Patentschriften 2 497 349, 3 287 423, 3 316 302, 3 927 108, 3 937 735, 3 946 076 und 4 042 630 verwiesen. Die Oxidationsreaktion wird vorzugsweise bei einer Temperatur von 120–190°C ausgeführt. Der Reaktionsdruck ist nicht kritisch, soll jedoch ausreichen, um im System eine flüssige Phase aufrechtzuerhalten. Der Umsetzungsgrad auf Basis des zugeführten Cyclohexans liegt vorzugsweise zwischen 1 und 12%. Als sauerstoffhaltiges Gas kann Luft oder mit einem Teil des Abgases verdünnte Luft verwendet werden. Andere sauerstoffhaltige Gase sind zwar brauchbar, aber weniger wirtschaftlich.

Aus dem anfallenden Reaktionsgemisch wird auf bekannte Weise, z.B. mittels Destillation, Cyclohexanon abgetrennt. Das Gemisch enthält auch Cyclohexanol, das in einer Dehydrierungszone zu Cyclohexanon und Wasserstoff dehydriert wird. Die Dehydrierung kann auf bekannte Weise in der Gasphase oder in der flüssigen Phase mit z.B. Zink- oder Kupferkatalysatoren ausgeführt werden. Der Kürze halber wird auf die Berichte des Stanford Research Institute Nr. 7 (1965), 275–306, 7A (1968), 87–103 und 7B (1976), 183–186, der britischen Patentschriften 739 263, 825 602 und 909 227 und der amerikanischen Patentschrift 2 524 566 verwiesen. Der auf diese Weise erhaltene Wasserstoff wird abgetrennt, das Cyclohexanon gewonnen und das unumgesetzte Cyclohexanol in die Dehydrierungszone zurückgeführt. Der Wasserstoff ist mit organischen Verunreinigungen, insbesondere Cyclohexanon und Cyclohexanol, verunreinigt und wird mit Benzol oder vorzugsweise mit Cyclohexan ausgewaschen, bevor er der Benzolhydrierungsanlage zugeführt wird. Wenn als Waschflüssigkeit Cyclohexan verwendet wird, wird als besonderer Vorteil erreicht, dass die dann abgetrennte Waschflüssigkeit ohne vorangehende Reinigung als Speisung für die Cyclohexanoxidation verwendet werden kann. Die vorhandenen Verunreinigungen beschleunigen sogar die Oxidationsreaktion, so dass ein grösserer Durchsatz durch den Oxidationsreaktor erreicht wird. Eine andere vorteilhafte Möglichkeit bei Verwendung von Cyclohexan als Waschflüssigkeit ist die Aufbereitung der benutzten Waschflüssigkeit zusammen mit dem Produktgemisch der Oxidationsreaktion, vorzugsweise mittels Destillation.

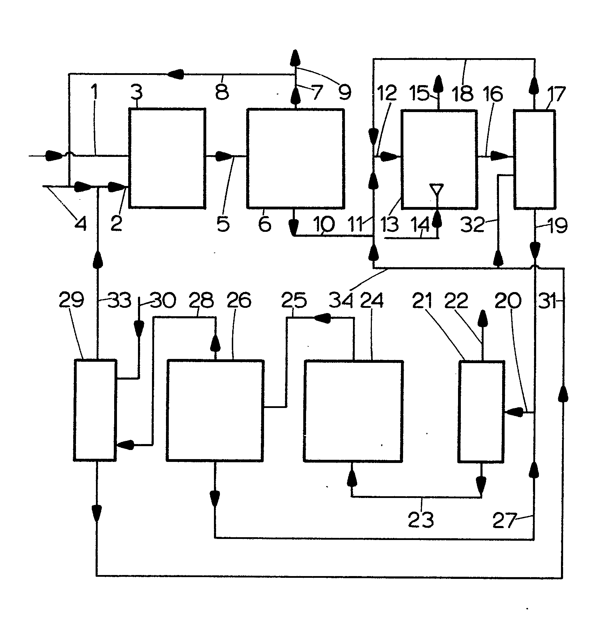

Es wird auf die beiliegende Figur verwiesen, die eine schematische Darstellung einer möglichen Ausführungsform des erfindungsgemässen Verfahrens zeigt.

Über Leitung 1 wird Benzoldampf und über Leitung 2 ein Gemisch von Wasserstoff und Stickstoff (80 Vol.-% Wasserstoff) in einen Hydrierungsreaktor 3 eingeleitet. Das Wasserstoff-Stickstoffgemisch wird durch Vermischung eines über Leitungen 4 und 8 zugeführten stickstoffreicheren Wasserstoff-Stickstoffgemisches mit über Leitung 33 zugeführtem Wasserstoffgas, das bei 10°C mit Cyclohexandampf gesättigt ist, erhalten. Reaktor 3 enthält Platin auf Aluminiumoxid (0,3 Gew.-% Platin) als Katalysator. Der Druck beträgt ca. 3 MPa und die Maximumtemperatur ca. 400°C. Wenn von schwefelhaltigem Benzol ausgegangen wird, kann die Hydrierung auf Wunsch in zwei Stufen mit zwischen diesen beiden Stufen einer Wasserstoffsulfid-Absorptionsstufe ausgeführt werden, wie dies in der britischen Patentschrift 1 104 275 beschrieben wird. Das austretende Gasgemisch strömt durch Leitung 5 zum Kondensator 6, in dem man Cyclohexan kondensieren lässt. Das über Leitung 7 entweichende Abgas wird teilweise über Leitungen 8 und 2 zum Hydrierungsreaktor 3 zurückgeführt und teilweise über Leitung 9 abgelassen. Das Abgas enthält ca. 22 Vol.-% Wasserstoff. Das Kondensat besteht aus nahezu reinem Cyclohexan mit einem Benzolgehalt von nur ca. 0,01 Gew.-% und einem Gehalt an anderen Verunreinigungen von weniger als 0,04 Gew.-% und wird über Leitungen 10, 11 und 12 in Oxidationsreaktor 13 eingeleitet. Hier wird das Cyclohexan in der flüssigen Phase mit über Leitung 14 zugeführter Luft und in Gegenwart von Kobaltnaphthenat als Katalysator bei ca. 160°C und einem Druck von ca. 0,9 MPa oxidiert. Die Oxidation kann auf Wunsch auch ohne Katalysator ausgeführt werden. Der Umsetzungsgrad des Cyclohexans beträgt ca. 4%. Aus dem über Leitung 15 entweichenden Abgas wird auf bekannte Weise mittels Kondensation Cyclohexan zurückgewonnen, das über eine nicht gezeichnete Leitung in Oxidationsreaktor 13 zurückgeführt wird. Das flüssige Reaktionsgemisch strömt über Leitung 16 zur Destillationskolonne 17, in der Cyclohexan abdestilliert wird. Dies wird über Leitungen 18 und 12 in Oxidationsreaktor 13 zurückgeführt. Das zurückbleibende Gemisch von Cyclohexanon und Cyclohexanol strömt über Leitungen 19 und 20 zur Destillationskolonne 21. Hier wird Cyclohexanon abgetrennt, das über Leitung 22 abgeführt wird. Cyclohexanol strömt, eventuell nach weiterer Reinigung, über Leitung 23 zum Dehydrierungsreaktor 24, wo es auf bekannte Weise mit Hilfe eines Kupfer- oder Zinkkatalysators dehydriert wird. Die Dehydrierung kann z.B. bei einer Temperatur von etwa 150–350°C und einem Druck von etwa 0,01–1 MPa ausgeführt werden, erfolgt aber vorzugsweise in der Gasphase bei einer Temperatur von ca. 250°C und einem Druck von 0,1 MPa. Das Reaktionsgemisch strömt über Leitung 25 zum Kondensator 26, in dem Cyclohexanon und unumgesetztes Cyclohexanol kondensiert werden. Das Cyclohexanon-Cyclohexanolgemisch wird über Leitungen 27 und 20 in Destillationskolonne 21 eingeleitet. Die aus verunreinigtem Wasserstoff bestehende Gasphase strömt über Leitung 28 zur Waschkolonne 29, in der sie mit über Leitung 30 zugeführtem Cyclohexan ausgewaschen wird. Die benutzte Waschflüssigkeit strömt über Leitungen 31, 34, 11 und 12 zum Oxidationsreaktor 13 oder wird gänzlich oder teilweise über die Leitungen 31 und 32 in Destillationskolonne 17 eingeleitet. Der gereinigte Wasserstoff strömt über Leitungen 33 und 2 zum Hydrierungsreaktor 3.

Die vorliegende Erfindung wird anhand der nachstehenden als Beispiele dienenden Versuche I und II und des Vergleichsversuchs A näher erläutert.

Beispiel Versuch I

Man lässt verunreinigtes Wasserstoffgas, das bei der Dehydrierung von Cyclohexanol zu Cyclohexanon und Wasserstoffgas anfällt, bei einer Temperatur von 10°C auskondensieren. Das übrigbleibende Wasserstoffgas enthält dann noch ca. 4 mg Cyclohexanon und ca. 0,6 mg Cyclohexanol je Liter Gas. Man leitet dieses Gas mit einer Geschwindigkeit von 32,5 l je Stunde unten in eine Waschkolonne mit einem Innendurchmesser von 15,5 mm und einer Länge von 365 mm ein, welche mit Glasperlen mit einem Durchmesser von 3 mm gefüllt ist. Oben in die Waschkolonne leitet man je Stunde 200 ml flüssiges Cyclohexan von 10°C ein. Die benutzte Waschflüssigkeit wird unten aus der Kolonne abgeführt. An der Oberseite wird gereinigtes Wasserstoffgas abgeführt.

Der auf diese Weise gereinigte Wasserstoff wird unter atmosphärischem Druck bei einer Bettemperatur von 160°C vermischt mit Benzol in einer Menge von 40 ml je Stunde durch ein Katalysatorbett mit 21,5 g (ca. 20 ml), 0,3 gewichtsprozentigem Platin auf Aluminiumoxid geleitet.

Unter diesen Reaktionsbedingungen beträgt die Benzolumsetzung, nachdem die Reaktion stabil geworden ist (nach etwa 5 Stunden Reaktionszeit), 61,7%. Nach 96stündiger Reaktionszeit beträgt die Benzolumsetzung 61,5%. Die Cyclohexanausbeute ist 99,8%.

Beispiel Versuch II

Der Versuch aus Beispiel I wird wiederholt, wobei jedoch weniger Cyclohexan als Waschflüssigkeit zugeführt wird, nämlich 25 ml je Stunde. Nachdem die Reaktion stabil geworden ist (nach etwa 5 Stunden Reaktionszeit), beträgt die Benzolumsetzung etwa 62%. Nach 100stündiger Reaktionszeit beträgt die Benzolumsetzung 56,5%. Die Aktivität des Katalysators hat somit etwas abgenommen. Die Cyclohexanausbeute ist 99,7%.

Vergleichsversuch A

Der in Beispiel I beschriebene Versuch wird wiederholt, wobei der bei der Dehydrierung von Cyclohexanol anfallende Wasserstoff jedoch nicht gereinigt wird. Die Benzolumsetzung nimmt nun in 97stündiger Reaktionszeit ab bis auf 45,4 Gew.-%. Die Katalysatoraktivität ist hiermit unzulässig niedrig geworden.

**Patentansprüche**

1. Verfahren zur Herstellung von Cyclohexanon, bei dem in einer Hydrierungszone Benzol in der Gasphase zu Cyclohexan hydriert wird, das Cyclohexan in einer Oxidationszone in der flüssigen Phase mit einem molekularen Sauerstoff enthaltenden Gas zu einem cyclohexanolhaltigen Gemisch oxidiert wird, das auf diese Weise erhaltene Cyclohexanol in einer Dehydrierungszone katalytisch zu Cyclohexanon und Wasserstoff dehydriert wird und Cyclohexanon und unumgesetztes Cyclohexanol abgetrennt werden, dadurch gekennzeichnet, dass das zurückbleibende, hauptsächlich aus Wasserstoff bestehende Gasgemisch mit Cyclohexan oder Benzol ausgewaschen und das Gas anschliessend in die Hydrierungszone eingeleitet wird.

2. Verfahren nach Anspruch 1, dadurch gekennzeichnet, dass in der Hydrierungszone ein Metall aus der Platingruppe als Katalysator verwendet wird.

3. Verfahren nach Anspruch 2, dadurch gekennzeichnet, dass in der Hydrierungszone ein Platinkatalysator verwendet wird.

4. Verfahren nach einem der Ansprüche 1–3, dadurch gekennzeichnet, dass als Waschflüssigkeit Cyclohexan verwendet wird und dass die benutzte Waschflüssigkeit in die Oxidationszone eingeleitet wird.

5. Verfahren nach einem der Ansprüche 1–3, dadurch gekennzeichnet, dass als Waschflüssigkeit Cyclohexan verwendet wird, die benutzte Waschflüssigkeit in eine Destillationszone eingeleitet wird, in die auch das aus der Oxidationszone stammende Reaktionsgemisch eingeleitet wird, und in dieser Destillationszone mittels Verdampfung Cyclohexan gewonnen wird.

6. Vorrichtung zum Ausführen des Verfahrens nach einem der Ansprüche 1–5, bestehend aus einem Gasphase-Hydrierungsreaktor mit Zufuhrleitungen für Benzol und Wasserstoff und einer Abfuhrleitung zu einem Flüssigphase-Oxidationsreaktor, welcher Oxidationsreaktor weiter versehen ist mit einer Zufuhrleitung für ein molekularen Sauerstoff enthaltendes Gas und einer Abfuhrleitung zu einem Dehydrierungsreaktor mit einer Vorrichtung zur Abtrennung von Cyclohexanon, einer Leitung zum Transport von verunreinigtem Wasserstoffgas von dieser Vorrichtung zu einer Gaswaschanlage, welche mit einer Zufuhrleitung für flüssiges Cyclohexan oder Benzol versehen ist, einer Abfuhrleitung für benutzte Waschflüssigkeit und einer Wasserstoffgasleitung zum Hydrierungsreaktor.

7. Vorrichtung nach Anspruch 6, dadurch gekennzeichnet, dass die Abfuhrleitung für benutzte Waschflüssigkeit mit dem Oxidationsreaktor verbunden ist.

8. Vorrichtung nach Anspruch 6, dadurch gekennzeichnet, dass die Abfuhrleitung für benutzte Waschflüssigkeit mit einer zwischen dem Oxidationsreaktor und dem Dehydrierungsreaktor geschalteten Destillationskolonne, bestimmt für das Abdestillieren von Cyclohexan, verbunden ist.

**Claims**

1. Process for the preparation of cyclohexanone, in which benzene is hydrogenated in the gaseous fase into cyclohexane in a hydrogenation zone, the cyclohexane is oxidized in the liquid phase with a gas containing molecular oxygen to form a mixture containing cyclohexanol in an oxidation zone, the resulting cyclohexanol is dehydrogenated catalytically into cyclohexanone and hydrogen in a dehydrogenation zone, and cyclohexanone and unconverted cyclohexanol are separated off, characterized in that the remaining gas mixture, which substantially consists of hydrogen, is washed with cyclohexane or benzene and then the gas is fed to the hydrogenation zone.

2. Process according to claim 1, characterized in that a metal from the platinum group is used as a catalyst in the hydrogenation zone.

3. Process according to claim 2, characterized in that a platinum catalyst is used in the hydrogenation zone.

4. Process according to any one of the claims 1–3, characterized in that the washing liquid used is cyclohexane and the effluent washing liquid is fed to the oxidation zone.

5. Process according to any one of the claims 1–3, characterized in that the washing liquid is cyclohexane, the effluent washing liquid is fed to a destillation zone to which the reaction mixture from the oxidation zone is also ved, and cyclohexane is recovered in this distillation zone by evaporation.

6. Installation for carrying out the process according to any one of the claims 1–5, comprising a hydrogenation reactor with feed conduits for benzene and hydrogen and a discharge conduit to a liquid-phase oxidation reactor that is furthermore provided with a feed line for a gas containing molecular oxygen and a discharge line to a dehydrogenation reactor with a cyclohexanone separating device, a conduit for contaminated hydrogen gas from this device to a gas washing device equipped with a feed line for liquid cyclohexane or benzene, a discharge line for effluent washing liquid and a hydrogen gas line to the hydrogenation reactor.

7. Installation according to claim 6, characterized in that the discharge line for effluent washing liquid runs to the oxidation reactor.

8. Installation according to claim 6, characterized in that the discharge line for effluent washing liquid runs to a distillation column for the distillation of cyclohexane arranged between the oxidation reactor and the dehydrogenation reactor.

## Revendications

1. Procédé de préparation de cyclohexanone, dans lequel on hydrogène dans une zone d'hydrogénation du benzène en phase gazeuse pour donner du cyclohexane, on oxyde le cyclohexane dans une zone d'oxydation en phase liquide avec un gaz contenant de l'oxygène moléculaire pour donner un mélange contenant du cyclohexanol, on déshydrogène catalytiquement le cyclohexanol obtenu de cette manière dans une zone de déshydrogénation pour donner de la cyclohexanone et de l'hydrogène et on sépare la cyclohexanone et le cyclohexanol n'ayant pas réagi, caractérisé en ce qu'on lave le mélange gazeux restant, constitué principalement d'hydrogène, avec du cyclohexane ou du benzène, puis en ce qu'on introduit le gaz dans la zone d'hydrogénation.

2. Procédé selon la revendication 1, caractérisé en ce qu'on utilise dans la zone d'hydrogénation un métal du groupe du platine comme catalyseur.

3. Procédé selon la revendication 2, caractérisé en ce qu'on utilise dans la zone d'hydrogénation un catalyseur au platine.

4. Procédé selon l'une des revendications 1–3, caractérisé en ce qu'on utilise comme liquide de lavage du cyclohexane et en ce qu'on introduit le liquide de lavage utilisé dans la zone d'oxydation.

5. Procédé selon l'une des revendications 1–3, caractérisé en ce qu'on utilise comme liquide de lavage du cyclohexane, en ce qu'on introduit le liquide de lavage utilisé dans une zone de distillation, dans laquelle on introduit également le mélange réactionnel provenant de la zone d'oxydation, et en ce qu'on recueille le cyclohexane dans cette zone de distillation par vaporisation.

6. Appareil pour réaliser le procédé selon l'une des revendications 1 à 5, constitué d'un réacteur d'hydrogénation en phase gazeuse avec des conduites d'amenée pour le benzène et l'hydrogène et avec une conduite d'évacuation vers un réacteur d'oxydation en phase liquide, réacteur d'oxydation muni en outre d'une conduite d'amenée pour un gaz contenant de l'oxygène moléculaire et d'une conduite d'évacuation vers un réacteur de déshydrogénation avec un dispositif de séparation de la cyclohexanone, une conduite pour le transport de l'hydrogène gazeux contenant des impuretés de ce dispositif vers une installation de lavage du gaz, munie d'une canalisation d'amenée pour le cyclohexane liquide ou la benzène, d'une canalisation d'évacuation pour le liquide de lavage utilisé et d'une canalisation d'hydrogène gazeux vers le réacteur d'hydrogénation.

7. Appareil selon la revendication 6, caractérisé en ce que la canalisation d'évacuation pour le liquide de lavage utilisé est reliée au réacteur d'oxydation.

8. Appareil selon la revendication 6, caractérisé en ce que la canalisation d'évacuation pour le liquide de lavage utilisé est reliée avec une colonne de distillation montée entre le réacteur d'oxydation et le réacteur de déshydrogénation, la colonne étant réglée pour la distillation du cyclohexane.